(19) Europäisches Patentamt
European Patent Office
Office européen des brevets

(11) Veröffentlichungsnummer: **0 249 103 A2**

# (12) EUROPÄISCHE PATENTANMELDUNG

(21) Anmeldenummer: **87107786.3**

(51) Int. Cl.4: **G01N 33/52**, C12Q 1/28

(22) Anmeldetag: **29.05.87**

(30) Priorität: **10.06.86 DE 3619436**

(43) Veröffentlichungstag der Anmeldung: **16.12.87 Patentblatt 87/51**

(84) Benannte Vertragsstaaten: **DE FR GB IT**

(71) Anmelder: **BAYER AG**
**Konzernverwaltung RP Patentabteilung**
**D-5090 Leverkusen 1 Bayerwerk(DE)**

(72) Erfinder: **Heidenreich, Holger, Dr.**
**Andreas-Gryphius Strasse 22**
**D-5000 Koeln 80(DE)**
Erfinder: **Wolfrum, Gerhard, Dr.**
**Domblick 17**
**D-5090 Leverkusen 3(DE)**
Erfinder: **Wehling, Klaus, Dr.**
**Am Rohm 121**
**D-5600 Wuppertal(DE)**
Erfinder: **Hugl, Herbert, Dr.**
**Gemarkenweg 9**
**D-5060 Bergisch Gladbach 2(DE)**

(54) **Triarly- und Trihetarylmethanderivate als Redoxindikatoren und deren Verwendung.**

(57) Die Erfindung betrifft Mittel zum Nachweis von Redoxreakionen enthaltend Verbindungen der allgemeinen Formel I

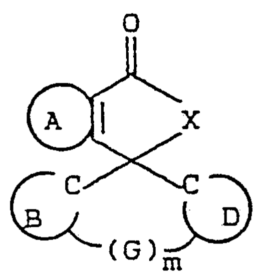

(I)

in der A, B, D, G, m und X die in der Beschreibung angegebene Bedeutung haben, Triaryl-und Trihetarylmethanderivate als Redoxindikatoren. Diese Redoxindikatoren können sehr gut zum Nachweis von Waserstoffperoxid eingesetzt werden insbesondere unter Zuhilfenahme von Peroxidasen oder von peroxidativ-aktiven Substanzen.



Xerox Copy Centre

## Triaryl-und Trihetarylmethanderivate als Redoxindikatoren und deren Verwendung

Die vorliegende Erfindung betrifft Mittel zum Nachweis von Redoxreaktionen enthaltend Triaryl-und Trihetarylmethanderivate als Redoxindikatoren. Diese Redoxindikatoren können sehr gut zum Nachweis von Wasserstoffperoxid eingesetzt werden insbesondere unter Zuhilfenahme von Peroxidasen oder von peroxidativ-aktiven Substanzen.

Weiterhin sind diese Redoxindikatoren geeignet zum Nachweis von Peroxidasen oder peroxidativ-aktiven Verbindungen, wobei auch andere Peroxide als Oxidationsmittel eingesetzt werden können.

Wasserstoffperoxid ist ein Reaktionsprodukt, das bei der enzymatisch katalysierten Oxidation von Substraten wie z.B. Glukose, Cholesterin, Harnsäure, Glycerin, Glycerinphosphat, Galaktose, Pyruvat oder auch Sarcosin durch eine entsprechende Oxidase wie Glukoseoxidase, Cholesterinoxidase, Uricase, Glycerinoxidase, Glycerinphosphatoxidase, Galaktoseoxidase, Pyruvatoxidase oder auch Sarcosinoxidase entsteht. Die genannten Substrate gehören zu der Gruppe von Analysensubstanzen die in der klinischchemischen Analytik eine Rolle spielen. Der Nachweis des bei der Oxidasereaktion gebildeten Wasserstoffperoxids kann polarographisch, titrimetrisch oder auch potentiometrisch erfolgen. Durch die Entdeckung von Wasserstoffperoxid umsetzenden Enzymen wie Peroxidase, Katalase oder Hämoglobin hat die kolorimetrische Bestimmung von Wasserstoffperoxid wesentlich an Bedeutung zugenommen. Peroxidasen wie auch peroxidativ-aktive Substanzen (z.B. Hämoglobin und Methämoglobin) katalysieren die Wasserstoffperoxid abhängige Oxidation von Indikatoren wie Guajakol, Dianisidinhydrochlorid oder auch ABTS zu farbigen Verbindungen. Eine der bekanntesten Nachweisreaktion für Wasserstoffperoxid ist die sogenannte "Trinder Reaktion" (Trinder, P. Ann. Clin. Biochem., Band 6 (1969), S. 24-27). In Anwesenheit von einer Peroxidase wird 4-Amino-antipyrin durch Wasserstoffperoxid oxidiert. Das Oxidationsprodukt ist in der Lage mit einem Phenol oder Phenolderivat zu kuppeln, wobei ein meist roter Chinoniminfarbstoff entsteht, dessen Konzentration photometrisch bestimmt werden kann.

Die vorliegende Erfindung betrifft Mittel zum Nachweis von Redoxreaktionen enthaltend Verbindungen der allgemeinen Formel I

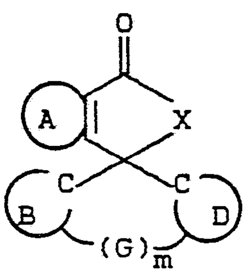

(I)

in der

A, B und D unabhängig voneinander für den Rest eines Aromaten oder Heteroaromaten,

G für O, $CH_2$ oder S;

m für die Zahl 0 oder 1 und

X für O, 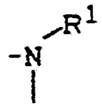 , 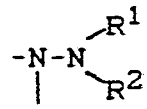 und

$-NR^1-NR^2-$stehen, wobei Aromaten die Reste Aryl oder Naphthyl, Heteroaromaten die Reste Pyridyl, Imidazolyl, Pyrazinyl und Indolyl sind, die ihrerseits in der Farbstoffchemie übliche Substituenten tragen können, $R^1$ und $R^2$ unabhängig voneinander Wasserstoff, Alkyl, Cycloalkyl, Aryl oder Aralkyl

bedeuten, die durch in der Farbstoffchemie übliche Substituenten substituiert sein können, oder $-NR^1R^2$ einen Pyrrolidin-, Pyrazolin-, Piperidin-, Piperazin-oder Morpholinrest, die durch in der Farbstoffchemie übliche Substituenten substituiert sein können, darstellt als Redoxindikatoren

In der Farbstoffchemie übliche Substituenten sind z.B. Halogen, Hydroxy, Alkoxy, Aryloxy, Aralkoxy, Aryl, Cycloalkyl, Hetaryl, Alkylmercapto, Arylmercapto, Alkylsulfonyl, Cyan, Alkylcarbonyl, Alkylcarbonyloxy, Nitro, Acylamino, Alkylsulfonsäure, Arylsulfonsäure, Alkylcarbonsäure, Aralkylcarbonsäure, Amino, das durch 1 oder 2 Alkyl-, Aryl-oder Aralkylgruppen substituiert sein kann, die ihrerseits wiederum durch Halogen, Cyan, Hydroxy, Sulfonsäure, Carbonsäure oder substituiertes Amino substituiert sein können, oder Aminogruppen, deren Substituenten ringgeschlossen sind.

Bevorzugt steht Alkyl für $C_1$-$C_{22}$-Alkyl, insbesondere für $C_1$-$C_{12}$-Alkyl und ganz besonders für $C_1$-$C_6$-Alkyl und Alkenyl für $C_2$-$C_5$-Alkenyl.

Unter Halogen ist im besonderen Fluor, Chlor und Brom zu verstehen.

Insbesondere werden unter Cycloalkyl, Cyclopentyl und Cyclohexyl, unter Aryl Phenyl und Naphthyl, unter Aralkyl Benzyl und Phenethyl, und unter Hetaryl Pyridyl, Pyrimidyl, Pyrazinyl, Triazinyl, Imidazolyl, Oxazolyl oder Thiazolyl verstanden.

Bevorzugtes Acyl ist $C_1$-bis $C_4$-Alkylcarbonyl und -sulfonyl und Benzoyl.

Die Verbindungen der allgemeinen Formel (I) sind als Farbbildner z.B. bei Durchschreibepapier oder Thermodruckpapier weitgehend bekannt (EP-A 108 383, EP-A 141 962). Beim Durchschreibepapier liegen die Farbbildner in gekapselter Form vor. Beim Schreiben werden die Kapseln zerstört und die Farbbildung der freigesetzten Farbbildner erfolgt beim Kontakt mit sauer modifizierter Tonerde.

Überraschenderweise wurde nun festgestellt, daß sich Verbindungen der allgemeinen Formel (I) auch sehr gut als Redoxindikatoren eignen. Besonders gut geeignet sind diese Verbindungen als Indikatoren zum qualitativen oder zum quantitativen Nachweis von Wasserstoffperoxid oder auch zum Nachweis von Peroxidasen bzw. peroxidativ-aktiven Substanzen. Die Oxidation der Indikatoren durch das Wasserstoffperoxid oder ein anderes Peroxid (z.B. Cumolhydroperoxid, Strontiumperoxid, 2,5-Dimethylhexan-2,5-dihydroperoxid, Diisopropylbenzoylhydroperoxid) kann durch die katalytische Wirkung einer Peroxidase oder einer peroxidativ-aktiven Substanz erfolgen. Geeignete Peroxidasen sind die aus Meerrettich, Kartoffeln oder die mikrobiologischen Ursprungs. Unter peroxidativ-aktiven Substanzen werden solche Substanzen verstanden die die Übertragung der Redoxäquivalente vom Wasserstoffperoxid oder einem anderen Peroxid auf die Indikatoren katalysieren wie z.B. Hämoglobin, Methämoglobin oder Myoglobin. Weiterhin sind die Verbindungen der allgemeinen Formel (I) geeignet um Oxidationsmittel wie z.B. Persulfat, Peracetat, Chloramin T oder auch Cyanoferri-Komplexe wie Kaliumhexacyanoferrat nachzuweisen.

Besonders gut eingesetzt werden können die Verbindungen der allgemeinen Formel (I) in Testmitteln für Substrate wie z.B. Glukose, Cholesterin, Harnsäure, Glycerin, Glycerinphosphat, Galaktose, Pyruvat oder auch Sarcosin die durch eine entsprechende Oxidase wie Glukoseoxidase, Cholesterinoxidase, Uricase, Glycerinoxidase, Glycerin phosphatoxidase, Galaktoseoxidase, Pyruvatoxidase oder Sarcosinoxidase in Anwesenheit von Sauerstoff unter Bildung von Wasserstoffperoxid oxidiert werden. Das gebildete Wasserstoffperoxid wird unter Verwendung der Verbindungen der allgemeinen Formel (I) nachgewiesen.

Wie schon angesprochen sind die Verbindungen der allgemeinen Formel (I) auch zum Nachweis von Peroxidasen oder peroxidativ-aktiven Substanzen geeignet. Als Testsysteme sind hier zu nennen der Nachweis von okultem Blut oder Peroxidase-markierte Immunteste.

Unter Testmittel oder Testsystem im Rahmen der vorliegenden Erfindung sind Z.B. solche zu verstehen, die in einer Küvette vermessen werden können. Die Testmittel enthalten neben den Redoxindikatoren der allgemeinen Formel (I) alle für den jeweiligen Parameternachweis notwendigen Reagenzien wie Enzyme, Substrate, Coenzyme, Effektoren, Antigene, Antikörper usw. Weiterhin können diese Testmittel noch nicht reagierende Substanzen wie z.B. Puffer, Netzmittel und Stabilisatoren enthalten. Aus den genannten Reagenzien und Substanzen können Reagenzkombinationen hergestellt werden, die als Lösung, als Pulver vermischt, als Tabletten oder als Lyophylisat vorliegen. Die Reagenzkombination (falls sie nicht schon als Lösung vorliegt) wird mit Wasser oder einem anderen geeigneten Lösungsmittel aufgenommen und zu einer Reagenzlösung bereitet. Besteht die Reagenzkombination aus einzelnen Komponenten so sind diese miteinander zu mischen. Nach Vermischen der Probe (z.B. Substratlösung, Enzymlösung, Blut, Serum, Plasma oder Urin) mit einem aliquoten Teil der Reagenzmischung wird die entstehende Farbe am Photometer vermessen und über den molaren Extinktionskoeffizienten und die zugesetzten Reagenz-bzw. Probevolumina die jeweilige Konzentration bzw. Substratkonzentration berechnet. Es sind sowohl kinetische als auch Endpunktsmessungen möglich.

Ebenso können die Verbindungen der allgemeinen Formel (I) zusammen mit Peroxidase oder einer peroxidativ-aktiven Substanz, dem/den für den jeweiligen Parameternachweis notwendigen Reagenzien bzw. anderen Enzymen, dem Puffersystem, gegebenenfalls Netzmitteln und Aktivatoren sowie anderen Hilfsstoffen auf saugfähigen Reagenzträgern wie Papieren, Vliesen etc. imprägniert werden. Dazu kann man eine oder mehrere Imprägnierlösungen in Form von wäßrigen oder organischen bzw. gemischten Lösungen herstellen, je nach dem wie sich die Reagenzien oder Hilfsstoffe lösen. Mit diesen Lösungen werden saugfähige oder quellfähige Träger, vorzugsweise Filterpapier oder saugfähige Glas-oder Kunststoffvliese, imprägniert oder besprüht. Anschließend wird getrocknet. Die so hergestellten Reagenzträger können als Schnelldiagnostica zur direkten Bestimmung von Inhaltsstoffen von Flüssigkeiten (z.B. in Körperflüssigkeiten wie Blut, Urin oder Speichel, oder in Lebensmitteln z.B. Fruchtsäften, Milch o.a.) eingesetzt werden. Die Flüssigkeit wird dabei direkt auf den Reagenzträger gebracht oder dieser kurz in die Flüssigkeit eingetaucht. Eine semiquantitative Bestimmung ist möglich, indem man die so entstandene Farbe einer Vergleichsfarbe zuordnet. Eine quantitative Auswertung läßt sich remissionsphotometrisch durchführen. Hierbei wirkt sich vorteilhaft aus, daß sich aus den Verbindungen der Formel (I) meist Farbstoffe entwickeln, die ihr Absorptionsmaximum im langwelligen Bereich des Spektrums haben. Für die Messung solcher Farbstoffe können dann Leuchtdioden als Lichtquelle eingesetzt werden.

Ebenso ist es möglich die Verbindungen der allgemeinen Formel (I) in Trägermatrices einzubringen, die aus Gießlösungen hergestellt wurde. Beispielhaft sind hier Cellulose, Celluosederivate, Gelatine, Gelatinederivate oder auch Kunststoffe wie Polyurethane und Acrylamid zu nennen. Dabei ist es vorteilhaft, wenn die Verbindungen der allgemeinen Formel (I) und gegebenenfalls die anderen notwendigen Reagenzien der Gießlösung direkt zugesetzt werden, wodurch es möglich wird, die Testvorrichtung bestehend aus Träger und Reagenzien in einem Arbeitsgang herzustellen.

Durch Eluieren der vorweggenannten Reagenzien mit Wasser bzw. Puffer oder Serum aus dem saugfähigen Träger läßt sich eine Reagenzlösung herstellen, mit der man wie oben beschrieben Substrate oder Enzyme in der Küvette am Photometer bestimmen kann.

Geeignete Puffer für die genannten Testmittel sind Phosphat-, Citrat-, Borat-, GOOD-Puffer mit Alkali- oder Ammoniumgegenionen. Andere Systeme sind jedoch ebenfalls brauchbar. Als pH-Werte sind 6 bis 10, insbesondere 6, 5 bis 7, 5 anzustreben.

Netzmittel sind insbesondere anionische und kationische Netzmittel, die mit den zwitterionischen erfindungsgemäßen Verbindungen ionische Wechselwirkungen eingehen. Nichtionogene Netzmittel, die die Enzyme aktivieren, sind jedoch ebenfalls brauchbar. Natriumlaurylsulfat, Dioctylnatriumsulfosuccinat und Alkylarylpolyetheralkohole werden bevorzugt.

Als Effektoren sind die für die jeweilige enzymatische Reaktion bekannten einzusetzen.

Als sonstige Hilfsstoffe können übliche Verdicker, Lösungsvermittler, Emulgatoren, optische Aufheller, Kontrastmittel etc. sinnvoll sein, wie sie in entsprechenden Testen mit anderen Chromogenen bekannt sind.

Von den Verbindungen der Formel (I) sind die Verbindungen der Formel (II)

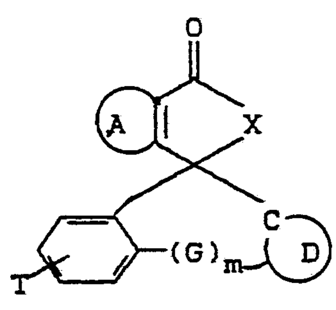

(II)

bevorzugt, worin

A gegebenenfalls substituiertes Phenyl, Pyridyl oder Imidazolyl bedeutet,

T Wasserstoff, Hydroxy, Alkyl, Aryl, Alkoxy, Phenoxy, $SO_3H$, -COOH oder

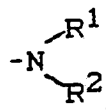

bedeutet,

wobei $R^1$ und $R^2$ die oben angegebene Bedeutung haben, und

X, G, D und m die oben angegebene Bedeutung haben.

Von besonderem Interesse sind Verbindungen der Formel (III)

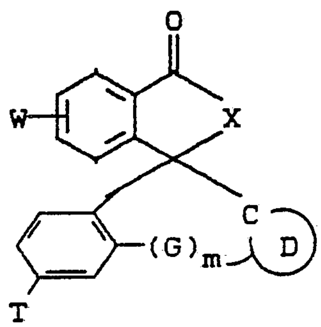

(III)

worin

W Wasserstoff,

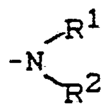,

Alkyl, Alkoxy oder Halogen,

T Wasserstoff, Alkyl, Alkoxy oder

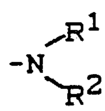,

X 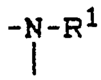, 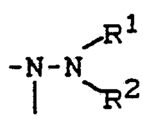

oder $-NH-NR^1-$,

D den Rest Phenyl, Naphthyl oder Indolyl bedeutet, und

G, $R^1$, $R^2$ und m die oben angegebene Bedeutung haben,

Ganz besonders bevorzugt sind Verbindungen der Formel (IV)

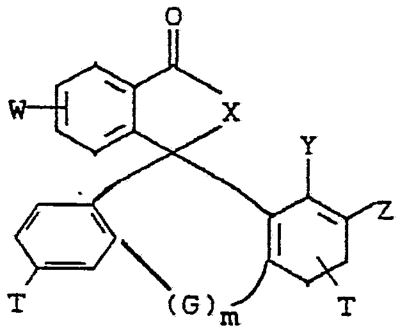

(IV)

worin

X 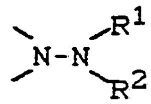

oder $-NH-NR^1$,

W Wasserstoff oder

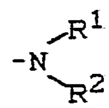 ,

T Wasserstoff, $C_1$-$C_4$-Alkyl, $C_1$-$C_4$-Alkoxy oder

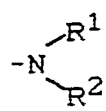

G 0,

m 0 oder 1 und

Y und Z Wasserstoff oder einen ankondensierten Benzoring und

$R^1$ und $R^2$ unabhängig voneinander Wasserstoff, $C_1$-$C_4$-Alkyl, das durch Halogen, Hydroxy, Cyano, $C_1$-$C_5$-Alkoxycarbonyl, $-SO_3H$ oder $-COOH$ substituiert sein kann, Aryl oder Aralkyl bedeuten,

oder

$NR^1$, $R^2$ ein Pyrrolidin, Piperidin oder Morpholin-Rest bedeutet.

Ganz besonders bevorzugt sind Verbindungen der Formel (V)

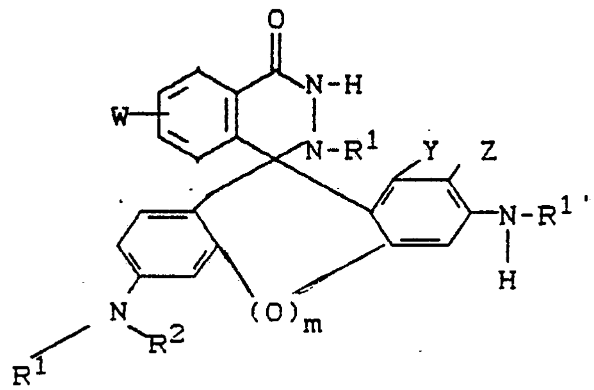

(V)

worin

W, $R^1$, $R^2$, Y, Z und m die oben angegebene Bedeutung haben.

Verfahren zur Herstellung der genannten Verbindungen sind in EP-A 141 962 und EP-A 108 382 beschrieben.

Mit den nachfolgenden Beispielen soll die vorliegende Erfindung näher beschrieben und illustriert werden.

Beispiel 1

4,15 g 3,3-Bis-(4-dimethylaminophenyl)-6-dimethylaminophthalid werden in 20 ml Ethanol mit 10 ml Hydrazinhydrat zum Rückfluß erhitzt. Nach 3 Stunden trägt man den Reaktionsansatz auf 100 ml Eiswasser aus und saugt ab. Das Rohprodukt der folgenden Konstitution

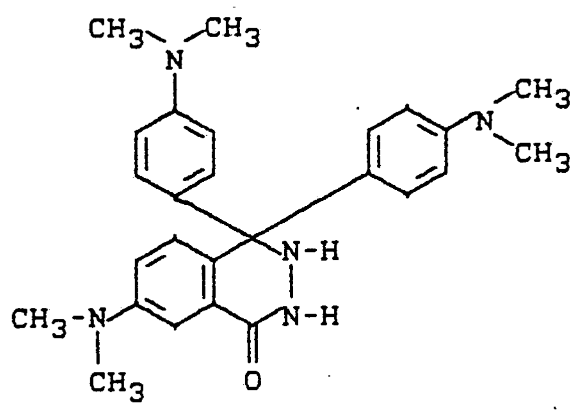

schmilzt bei 263°C.

Beispiel 2

4,15 g 3,3-Bis-(4-dimethylaminophenyl)-6-dimethylaminophthalid werden mit 20 ml Diethanolamin für 2 Stunden auf 150°C erhitzt. Man läßt abkühlen, trägt die Lösung auf Eiswasser aus und saugt den gelblichen Niederschlag ab. Nach Umkristallisation aus Ethanol schmilzt die Substanz der folgenden Konstitution

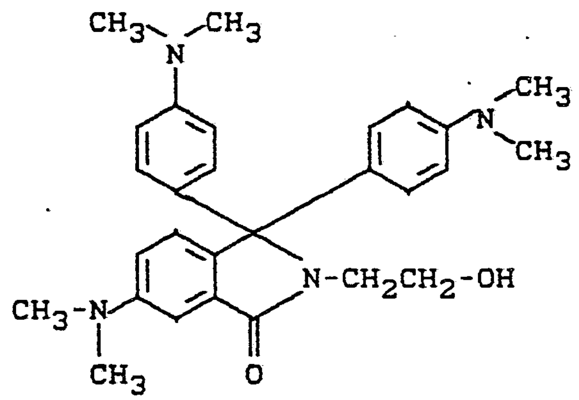

bei 252°C.

Farbe des Oxidationsprodukts: gelborange

Beispiel 3

31,8 g 3,3-Bis-(4-hydroxyphenyl)-1(3H)-isobenzofuranon werden in 150 ml Ethanol mit 30 ml Hydrazinhydrat zum Rückfluß erhitzt. Nach 5 Stunden gibt man die Mischung auf Eis und säuert mit Essigsäure an. Der abgesaugte Niederschlag wird aus Ethanol umkristallisiert. Die bei 279°C schmelzende Substanz hat die folgende Formel

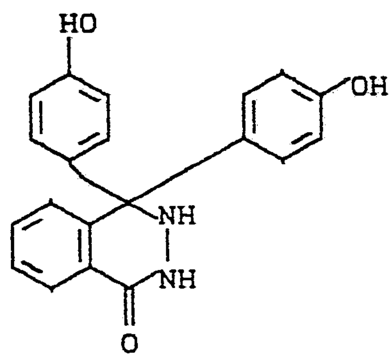

Farbe des Oxidationsprodukts: orange

Beispiel 4

15,7 g 2-(2-Hydroxy-4-diethylamino)benzoyl-benzoesäure werden bei einer Temperatur von 8 bis 12°C in 62 ml Schwefelsäuremonohydrat eingetragen. Anschließend gibt man 9,95 g 4-Methoxy-diphenylamin hinzu und läßt 2 Tage bei Raumtemperatur rühren. Danach trägt man die Reaktionslösung auf Eis aus, stellt mit Natronlauge auf pH 11, überschichtet mit 300 ml Toluol und erhitzt 3 Stunden zum Rückfluß. Anschließend trennt man die Toluolphase ab und dampft sie ein. Nach Behandlung mit A-Kohle kristallisiert die folgende Substanz aus Toluol mit dem Schmelzpunkt 194°C.

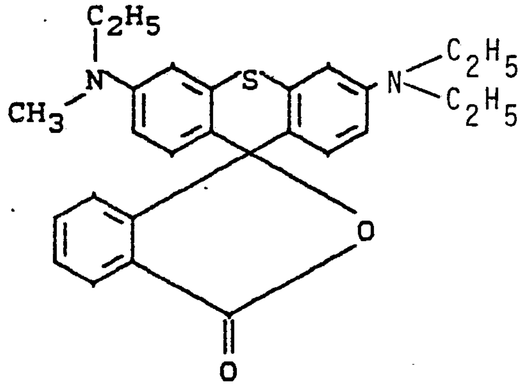

Farbe des Oxidationsprodukts: grauviolett.

Beispiel 5

4,62 g 3-Diethylamino-7-anilinofluoran werden 1 Stunde in 25 ml Ethylglykol mit 6 ml Hydrazinhydrat zum Rückfluß erhitzt. Nach dem Abkühlen tropft man Eiswasser hinzu und saugt den Niederschlag ab. Die Verbindung der folgenden Konstitution schmilzt bei 146°C.

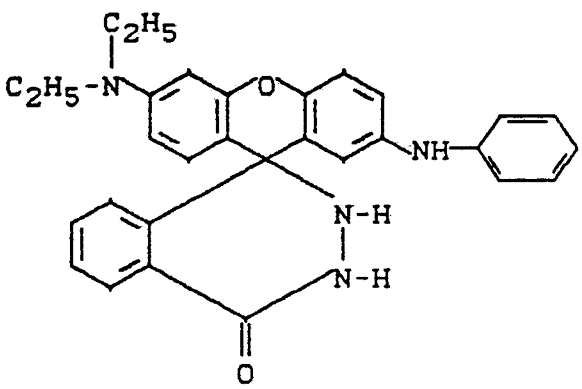

Die nachfolgend genannten Verbindungen werden analog hergestellt.

Beispiel 6 *orange

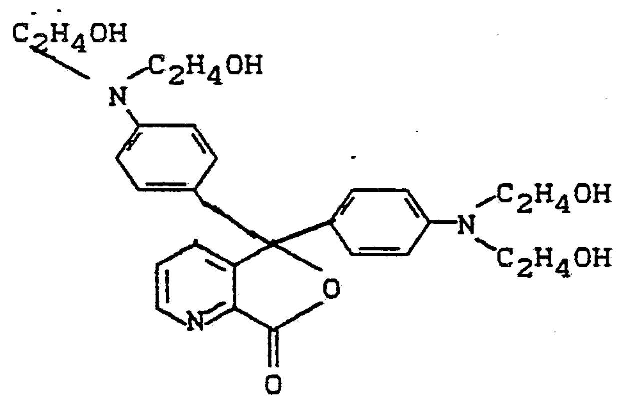

Beispiel 7 *orange

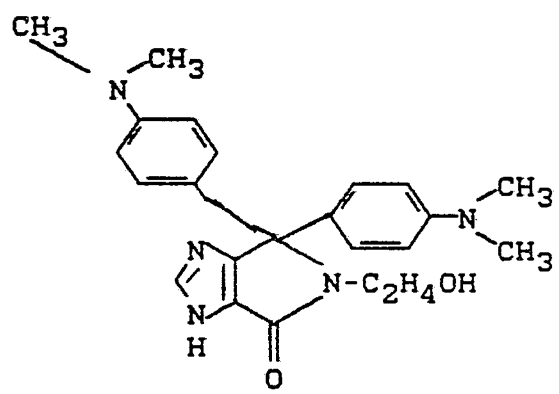

Beispiel 8 *gelb

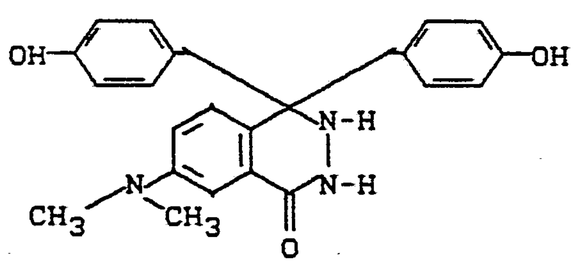

* = Farbe des Oxidationsproduktes

Beispiel 9 *gelborange

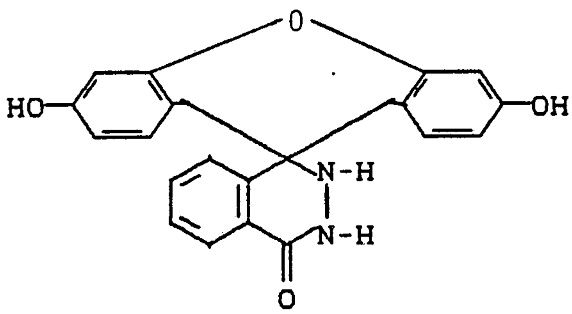

Beispiel 10 *gelborange

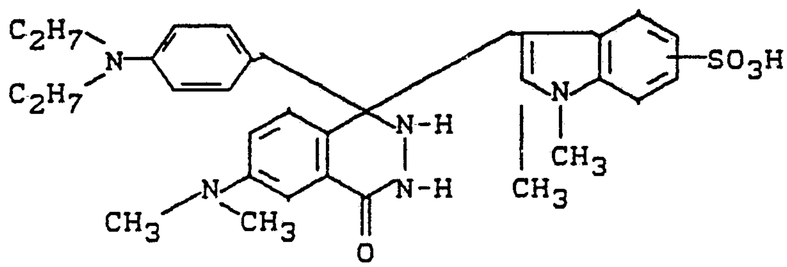

Beispiel 11 *rotorange

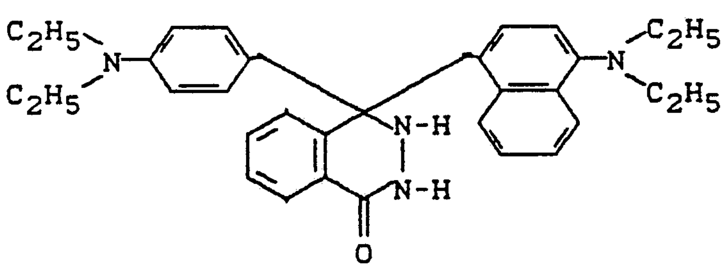

Beispiel 12 *orange

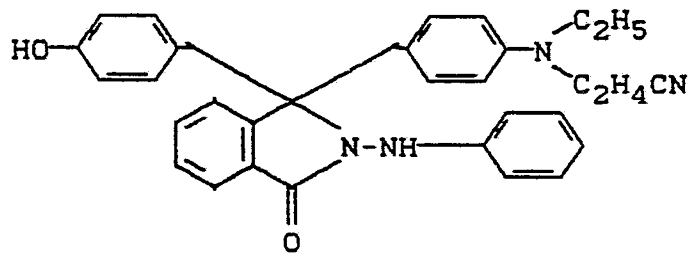

Beispiel 13 *rotorange

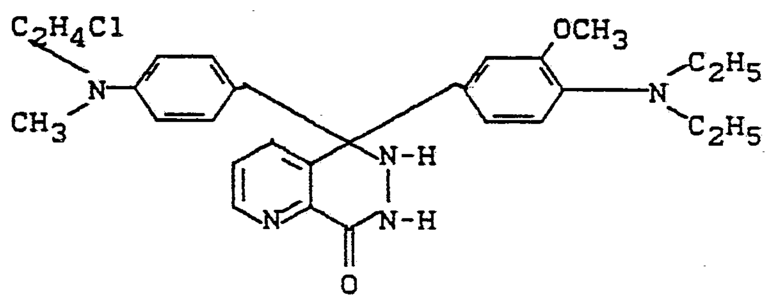

Beispiel 14 *gelborange

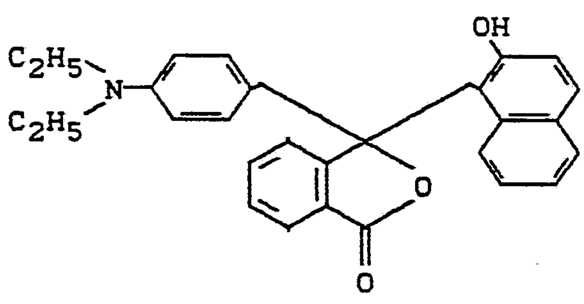

Beispiel 15 *gelb

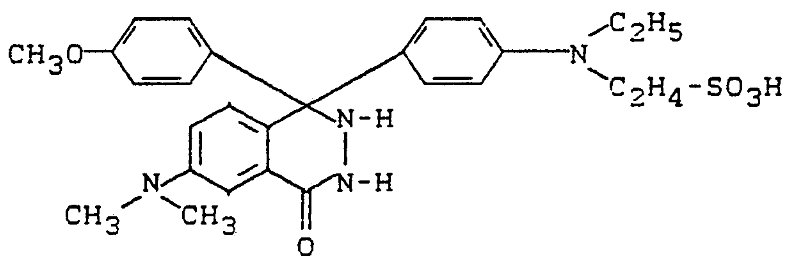

Beispiel 16 *orange

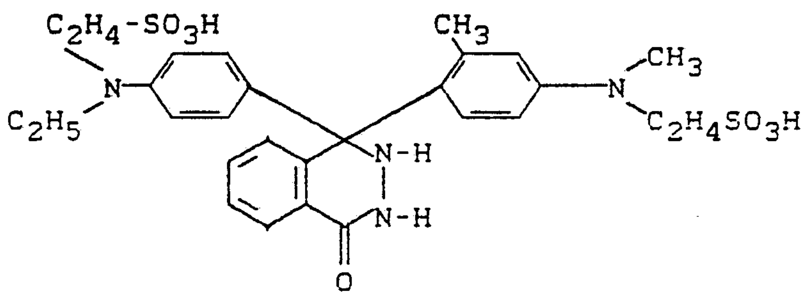

Beispiel 17 *orange

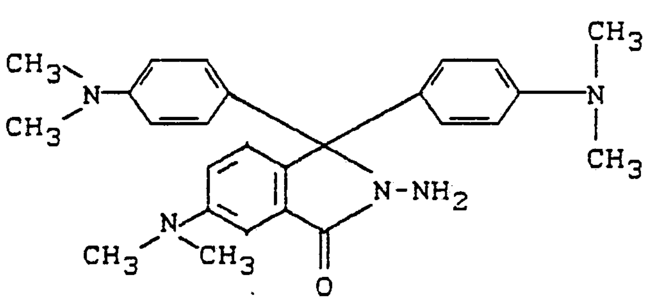

Beispiel 18 *gelb

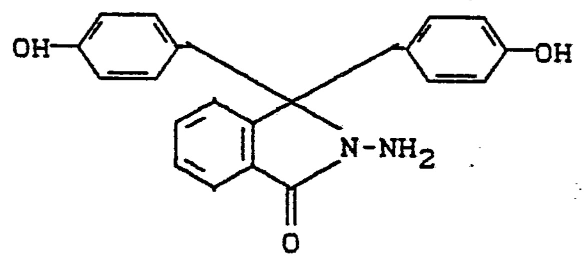

Beispiel 19 *orange

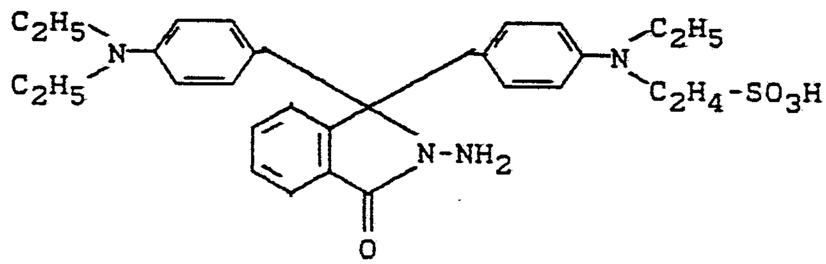

Beispiel 20 *rot

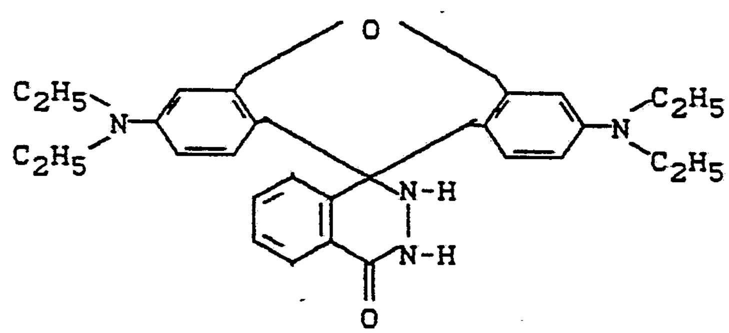

Beispiel 21 *rotbraun

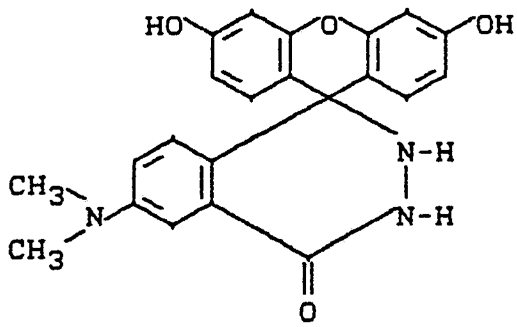

Beispiel 22 *rot

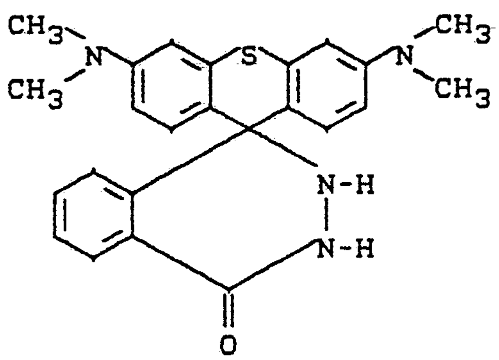

Beispiel 23 *blaugrün

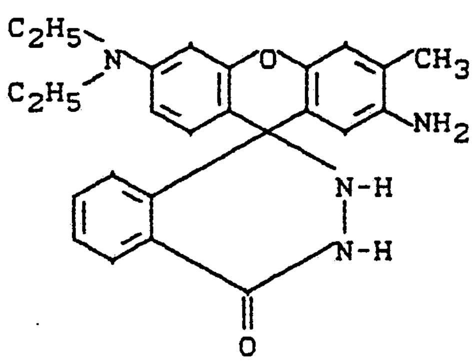

<u>Beispiel 24</u> *grün

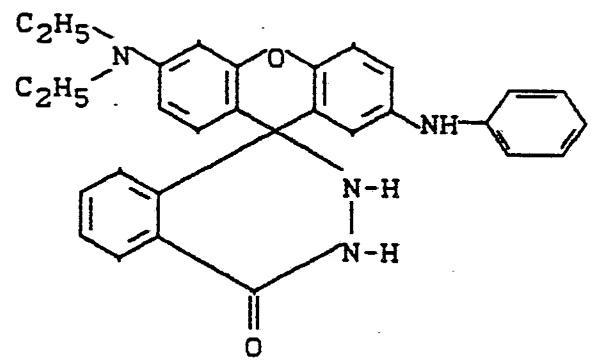

<u>Beispiel 25</u> *rot

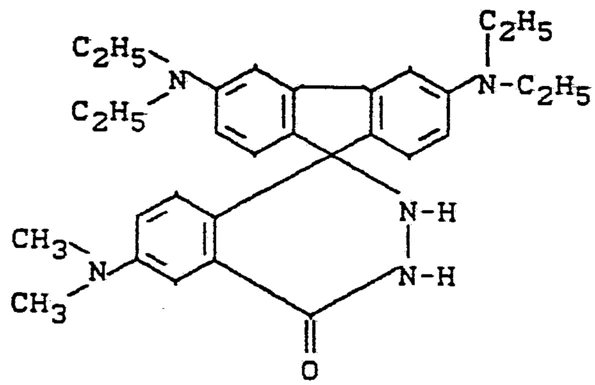

<u>Beispiel 26</u> *graugrün

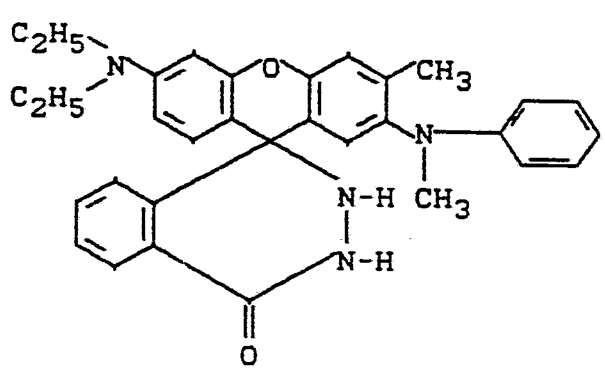

Beispiel 27 *graublau

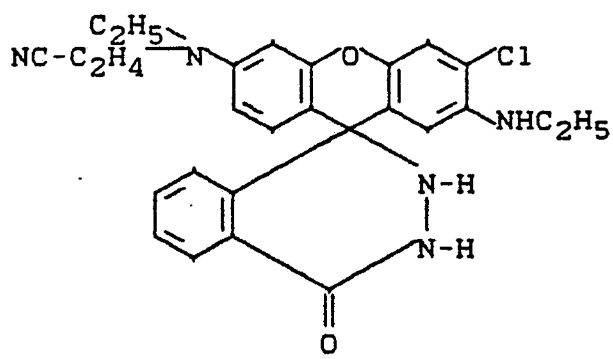

Beispiel 28 grauviolett

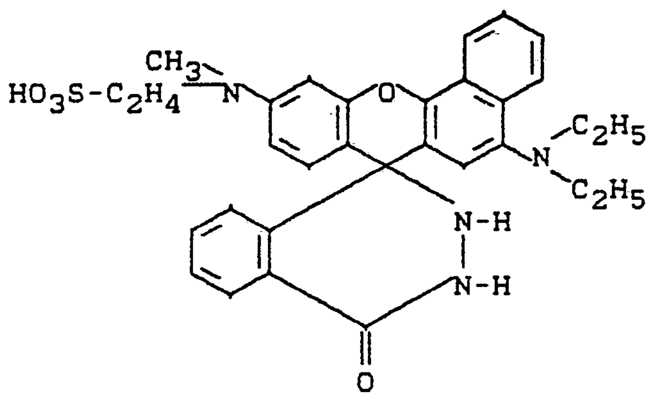

Beispiel 29 *blaugrau

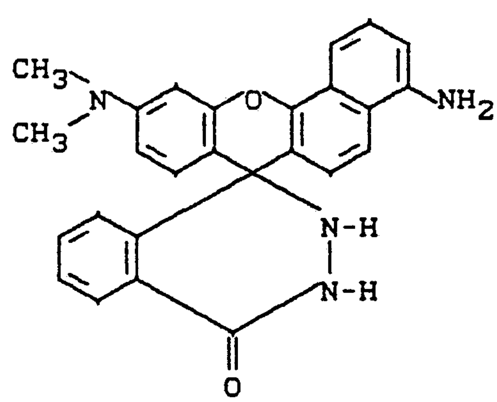

Beispiel 30 *graublau

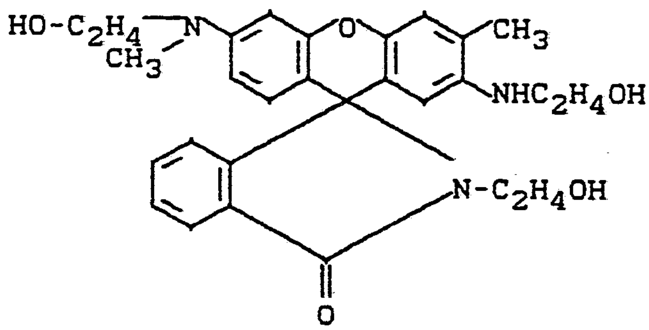

Beispiel 31 *graugrün

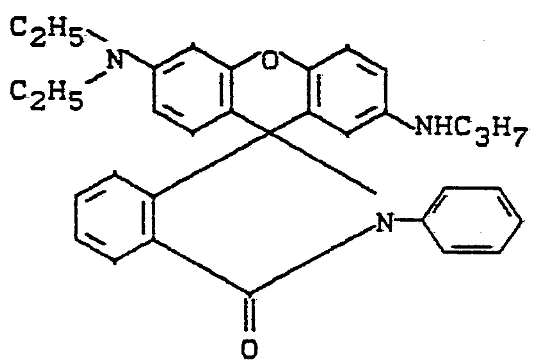

Beispiel 32 *violettgrau

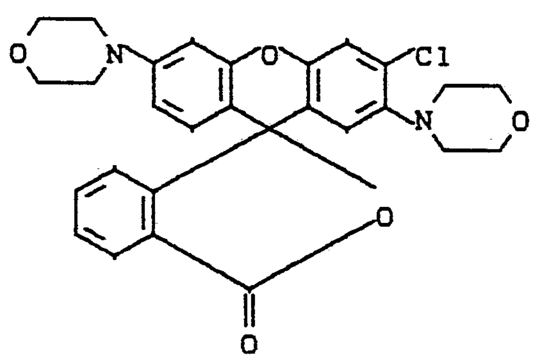

<u>Beispiel 33</u> *rot

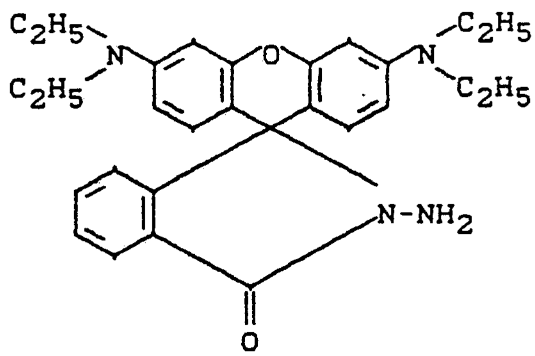

<u>Beispiel 34</u> *rotorange

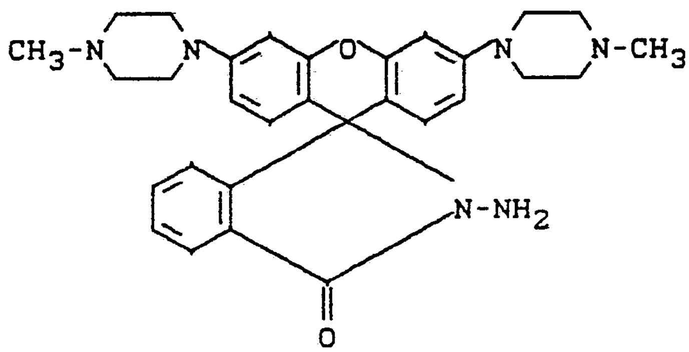

<u>Beispiel 35</u> *violettrot

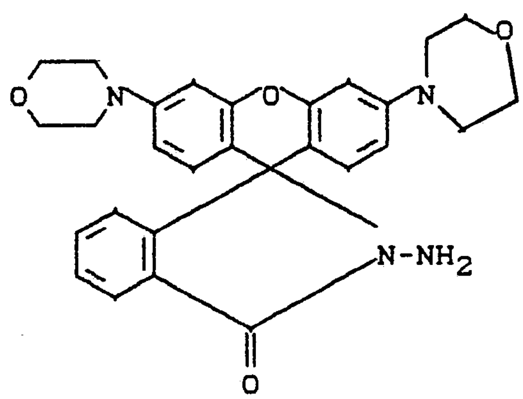

Beispiel 36 *graublau

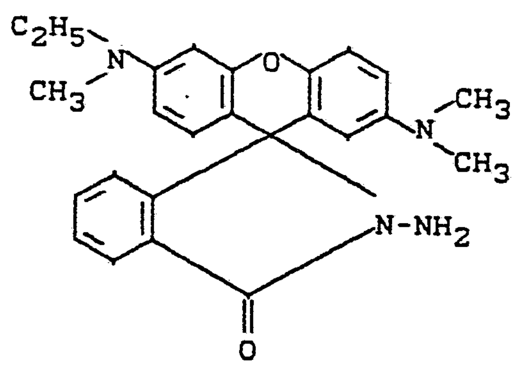

Beispiel 37 *rot

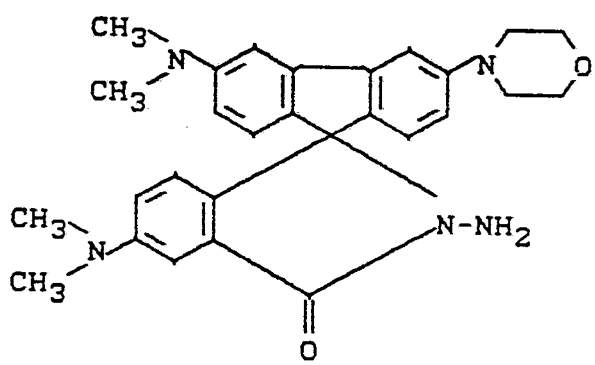

Beispiel 38 *grünviolett

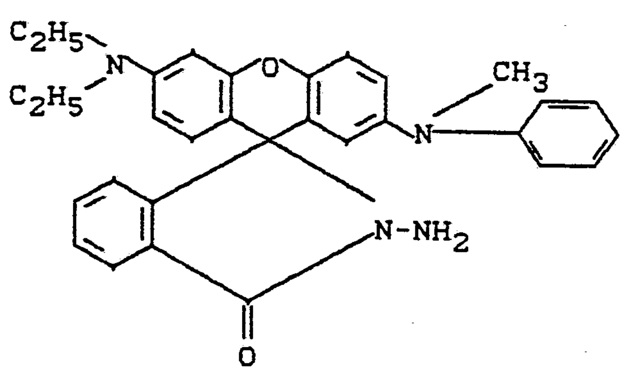

Beispiel 39 *pink

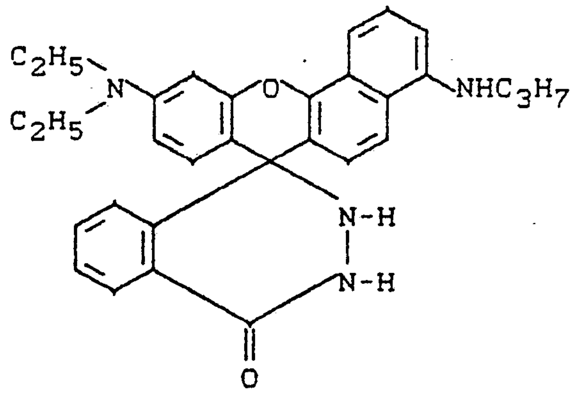

Beispiel 40

Zur Prüfung der Verbindung aus dem Beispiel 1 als Indikator in einem $H_2O_2$/Peroxidase-Testsystem wurde diese Verbindung in einer Konzentration von 5 mM in DMF gelöst. Diese Lösung wurde dann 1 + 1 mit Puffer (Citrat 0,1 M/l, pH 4,55, Tris 0,1 M/l, pH 6,57 und pH 7,8 gemischt und 5 µl Peroxidase (500 k U/l) wurden zu 0,5 ml dieser Lösung zugesetzt. Nach Zugabe von 10 µl $H_2O_2$ (3, 4 mM/l ≙ $E_{240}$ = 0,168) und einer Inkubationszeit von 5 min wurden die Extinktionen gemessen. Wie die Abb. 1 zeigt, verschiebt sich das Absorptionsmaximum in Abhängigkeit vom pH-Wert.

Die Kurve 1 zeigt das Absorptionsspektrum bei pH 6,57 und die Kurve 2 bei pH 4,6.

Die Tabelle 1 zeigt die gemessenen Extinktionen bei den verschiedenen Wellenlängen und pH-Werten.

Tabelle 1

| pH-Wert | Farbe | Absorptions-maximum (nm) | Extinktions-differenz/5 min |
|---|---|---|---|
| 4,55 | gelb | 415 | 1,707 |
| 6,57 | gelb-orange | 490 | 1,351 |
| 7,80 | gelb-orange | 490 | 1,803 |

Zur Prüfung der Funktion und Linearität wurden 0,5 ml der obigen Lösung mit Tris Puffer pH 7,8 mit 5 µl Peroxidase (500 k U/l) und 5 µl $H_2O_2$ verschiedener Konzentration versetzt. Die Tabelle 2 zeigt die Resultate im Vergleich zum "Trinder-Farbsystem" 4-Aminoantipyrin-Dichlor-2-hydroxybenzolsulfonsäure.

Tabelle 2

Extinktionsdifferenz/5 Minuten

| | $H_2O_2$-Konzentration (Ext. bei 240 nm) | | | |
|---|---|---|---|---|
| | 0,021 | 0,045 | 0,083 | 0,168 |
| Verbindung aus Bsp.1 | 0,116 | 0,229 | 0,419 | 0,888 |
| 490 nm | 0,114 | 0,225 | 0,441 | 0,910 |
| DCHBS/Aminopy. | 0,139 | 0,274 | 0,546 | 1,076 |
| 510 nm | 0,144 | 0,273 | 0,534 | 1,103 |

Die Tabelle 3 zeigt die Resultate der Prüfung auf Störungen durch Ascorbinsäure. Für diese Prüfung werden dem obigen Ansatz 5 µl einer $H_2O_2$-Lösung ($H_2O_2$ Konzentration in der Probe = $E_{240}$ = 0,242) und noch 5 µl einer Ascorbinsäurelösung von 1 mM/l zugesetzt und die resultierende Extinktion gemessen.

Tabelle 3

| ohne Ascorbinsäure | $E_1$ | $E_2$ | $\Delta E_{490}$ nm | |
|---|---|---|---|---|
| | 0,694 | 1,963 | 1,269 | |
| | 0,720 | 1,992 | 1,272 | |
| | 0,709 | 2,002 | 1,293 | $\bar{x}$ = 1,278 |
| mit Ascorbinsäure | 0,585 | 1,899 | 1,314 | |
| | 0,573 | 1,859 | 1,286 | |
| | 0,577 | 1,854 | 1,277 | $\bar{x}$ = 1,292 |

Die gemessenen Werte in Anwesenheit von Ascorbinsäure liegen im Mittel um 1,1% über den Werten die in Abwesenheit der Ascorbinsäure gemessen wurden. Der vorliegende Test wird also durch Ascorbinsäure nicht beeinflußt.

Beispiel 41

Prüfung der Verbindung aus Beispiel 5

Ansatz:
1000 µl Puffer (Citrat 0,1 M/l pH 5, Tris 0,1 M/l pH 7 und pH 9)
900 µl DMF
100 µl Verb. Beispiel 5, 2,02 mM in DMF
20 µl POD (1000 kU/l)
20 µl $H_2O_2$ Lösung

Das gemessene Absorptionsmaximum liegt bei 720 nm. Die Extinktionsdifferenzen bei verschiedenen pH-Werten sind in der Tabelle 4 aufgelistet. Die Probenkonzentration betrug 5 mM/l an $H_2O_2$.

Tabelle 4

| | $\Delta E_{720}$ nach 5 min |
|---|---|
| pH 5 | 0,110 |
| pH 7 | 0,185 |
| pH 9 | 0,040 |

Die Extinktionsabnahme innerhalb von 20 min (Farbstabilität) beträgt 0,8 %. Die Prüfung der Störung durch Ascorbinsäure wurde analog Beispiel 40 durchgeführt. Die Tabelle 5 zeigt die Resultate.

Tabelle 5

| | $E_1$ | $E_2$ | $\Delta E_{720}$ nm | |
|---|---|---|---|---|
| ohne Ascorbinsäure | 0,138 | 0,374 | 0,236 | |
| | 0,148 | 0,380 | 0,232 | |
| | 0,140 | 0,375 | 0,235 | $\bar{x}$ = 0,234 |
| mit Ascorbinsäure | 0,107 | 0,349 | 0,242 | |
| | 0,107 | 0,353 | 0,246 | |
| | 0,103 | 0,358 | 0,255 | $\bar{x}$ = 0,248 |

In Anwesenheit von Ascorbinsäure liegen die Meßwerte im Mittel um 5,7 % höher.

Die Tabelle 5 zeigt die Linearitäts-und Funktionsprüfung. Dem obigen Testansatz wurden $H_2O_2$-Lösungen unterschiedlicher Konzentrationen zugesetzt und die Extinktion nach 5 min gemessen.

Tabelle 6

| $[H_2O_2]$-Probe | Verbindung Beispiel 5 | | | Vergleich 4-AAP / DCHBS (Trinder) | | |
|---|---|---|---|---|---|---|
| $E_{240}$ | pH 7 | | 720 nm | pH 7,5 | | 510 nm |
| | $E_1$ | $E_2$ | $\Delta E_{720}$ | $E_1$ | $E_2$ | $\Delta E_{510}$ |
| 0,072 | 0,146 | 0,204 | 0,058 | 0,010 | 0,321 | 0,311 |
| 0,099 | 0,139 | 0,248 | 0,109 | 11 | 0,714 | 0,703 |
| 0,151 | 0,139 | 0,275 | 0,136 | 10 | 1,040 | 1,030 |
| 0,200 | 0,140 | 0,333 | 0,193 | 15 | 1,402 | 1,387 |
| 0,258 | 0,148 | 0,371 | 0,223 | 10 | 1,703 | 1,693 |
| 0,303 | 0,139 | 0,400 | 0,261 | 11 | 1,976 | 1,965 |
| 0,371 | 0,138 | 0,418 | 0,280 | 12 | 2,270 | 2,258 |
| 0,413 | 0,139 | 0,432 | 0,293 | 12 | 2,405 | 2,393 |
| 0,460 | 0,148 | 0,440 | 0,292 | 11 | 2,678 | 2,667 |
| 0,512 | 0,139 | 0,443 | 0,304 | 10 | 2,771 | 2,781 |

## Ansprüche

1. Mittel zum Nachweis von Redoxreaktionen enthaltend Verbindungen der allgemeinen Formel (I)

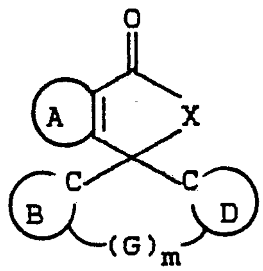

(I)

in der die Ringe

A, B und D unabhängig voneinander für den Rest eines gegebenenfalls substituierten Aromaten oder Heteroaromaten,

G für O, $CH_2$ oder S,

m für die Zahl 0 oder 1 und

X für O, 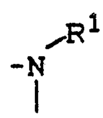 , 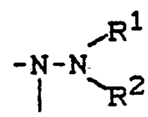 und

-$NR^1$-$NR^2$-stehen, wobei Aromaten die Reste Aryl oder Naphthyl, Heteroaromaten die Reste Pyridyl, Imidazolyl, Pyrazinyl und Indolyl sind, die ihrerseits in der Farbstoffchemie übliche Substituenten tragen können, $R^1$ und $R^2$ unabhängig voneinander Wasserstoff, Alkyl, Cycloalkyl, Aryl oder Aralkyl bedeuten, die durch in der Farbstoffchemie übliche Substituenten substituiert sein können, oder -$NR^1R^2$ gemeinsam einen Pyrrolidin-, Pyrazolin-, Piperidin-, Piperazin-oder Morpholinrest darstellen, die durch in der Farbstoffchemie übliche Substituenten substituiert sein können,

als Redoxindikatoren.

2. Mittel gemäß Anspruch 1 enthaltend Verbindungen der allgemeinen Formel (II)

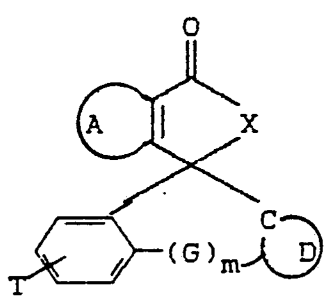

(II)

worin

A gegebenenfalls substituiertes Phenyl, Pyridyl oder Imidazolyl bedeutet,

T Wasserstoff, Hydroxy, Alkyl, Aryl, Alkoxy, Phenoxy, $SO_3H$, -COOH oder

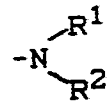

bedeutet,
wobei $R^1$ und $R^2$ die oben angegebene Bedeutung haben, und

X, G, D und m die oben angegebene Bedeutung haben, als Redoxindikatoren.

3. Mittel gemäß den Ansprüchen 1 und 2 enthaltend Verbindungen der allgemeinen Formel (III)

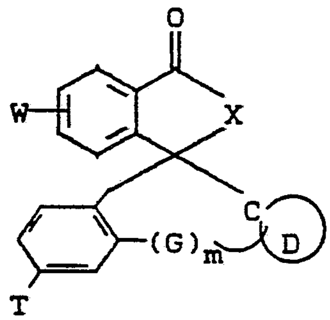

(III)

worin

W Wasserstoff,

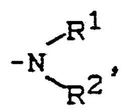

Alkyl, Alkoxy oder Halogen,

T Wasserstoff, Alkyl, Alkoxy oder

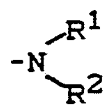

,

X 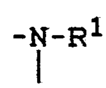 , 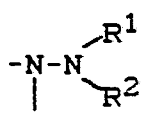

oder -NH-$NR^1$-,

D den Rest Phenyl, Naphthyl oder Indolyl bedeutet, und

G und m die oben angegebene Bedeutung haben.

4. Mittel gemäß den Ansprüchen 1 bis 3 enthaltend Verbindungen der allgemeinen Formel (IV)

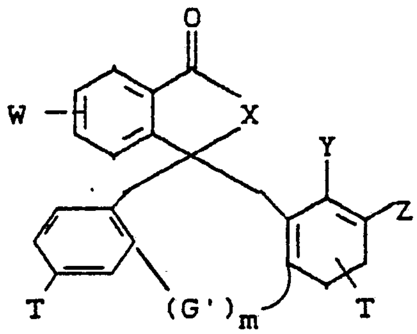

(IV)

worin

X 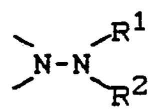

oder -NH-NR[1'],

W Wasserstoff oder

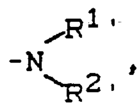

T Wasserstoff, $C_1$-$C_4$-Alkyl, $C_1$-$C_4$-Alkoxy oder

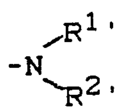

G 0,

m 0 oder 1 und

Y und Z Wasserstoff oder einen ankondensierten Benzoring und

$R^1$ und $R^2$ unabhängig voneinander Wasserstoff, $C_1$-$C_4$-Alkyl, das durch Halogen, Hydroxy, Cyano, $C_1$-$C_5$-Alkoxycarbonyl, $-SO_3H$ oder -COOH substituiert sein kann, Aryl oder Aralkyl bedeuten,

oder

$NR^1R^2$ ein Pyrrolidin, Piperidin oder Morpholin-Rest bedeutet,

als Redoxindikatoren.

5. Mittel gemäß den Ansprüchen 1 bis 4 enthaltend eine Verbindung der allgemeinen Formel (V)

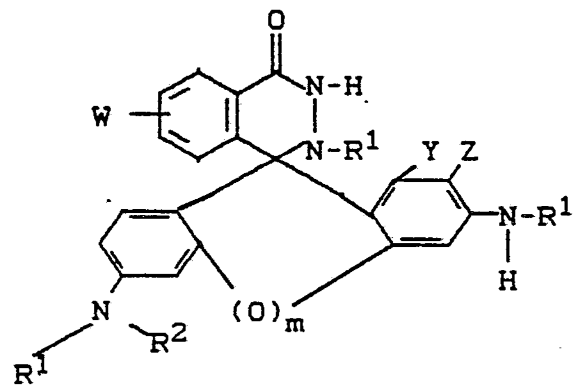

(V)

worin

W für Wasserstoff oder

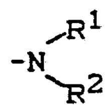

steht,

$R^1$ und $R^2$ unabhängig voneinander Wasserstoff, $C_1$-$C_4$-Alkyl, das durch Halogen, Hydroxy, Cyano, $C_1$-$C_5$-Alkoxycarbonyl, $-SO_3H$ oder -COOH substituiert sein kann, Aryl oder Aralkyl bedeuten und

Y, Z und m die oben angegebene Bedeutung haben,

als Redoxindikatoren.

6. Mittel gemäß den Ansprüchen 1 bis 5, die zusätzlich eine Peroxidase oder eine peroxidativ aktive Substanz enthalten.

7. Mittel gemäß den Ansprüchen 1 bis 6, die zusätzlich noch Puffer, Effektoren, Netzmittel und/oder Stabilisatoren enthalten.

8. Reagenz zum Nachweis einer Analysensubstanz enthaltend das Mittel gemäß den Ansprüchen 1 bis 7 und eine Oxidase.

9. Testvorrichtung zum Nachweis von Redoxreaktionen bestehend aus einem Träger enthaltend das Mittel gemäß den Ansprüchen 1 bis 7.

10. Testvorrichtung zum Nachweis einer Analysensubstanz bestehend aus einem Träger enthaltend das Reagenz gemäß Anspruch 8.

11. Verfahren zum Nachweis von Redoxreaktionen, dadurch gekennzeichnet, daß man eine Probe mit dem Mittel gemäß den Ansprüchen 1 bis 7 oder der Testvorrichtung gemäß Anspruch 9 in Kontakt bringt und die entstehende Reaktion verfolgt.

12. Verfahren zum Nachweis einer Analysensubstanz, dadurch gekennzeichnet, daß man eine Probe mit dem Reagenz gemäß Anspruch 8 oder mit der Testvorrichtung gemäß Anspruch 10 in Kontakt bringt und die entstehende Reaktion verfolgt.

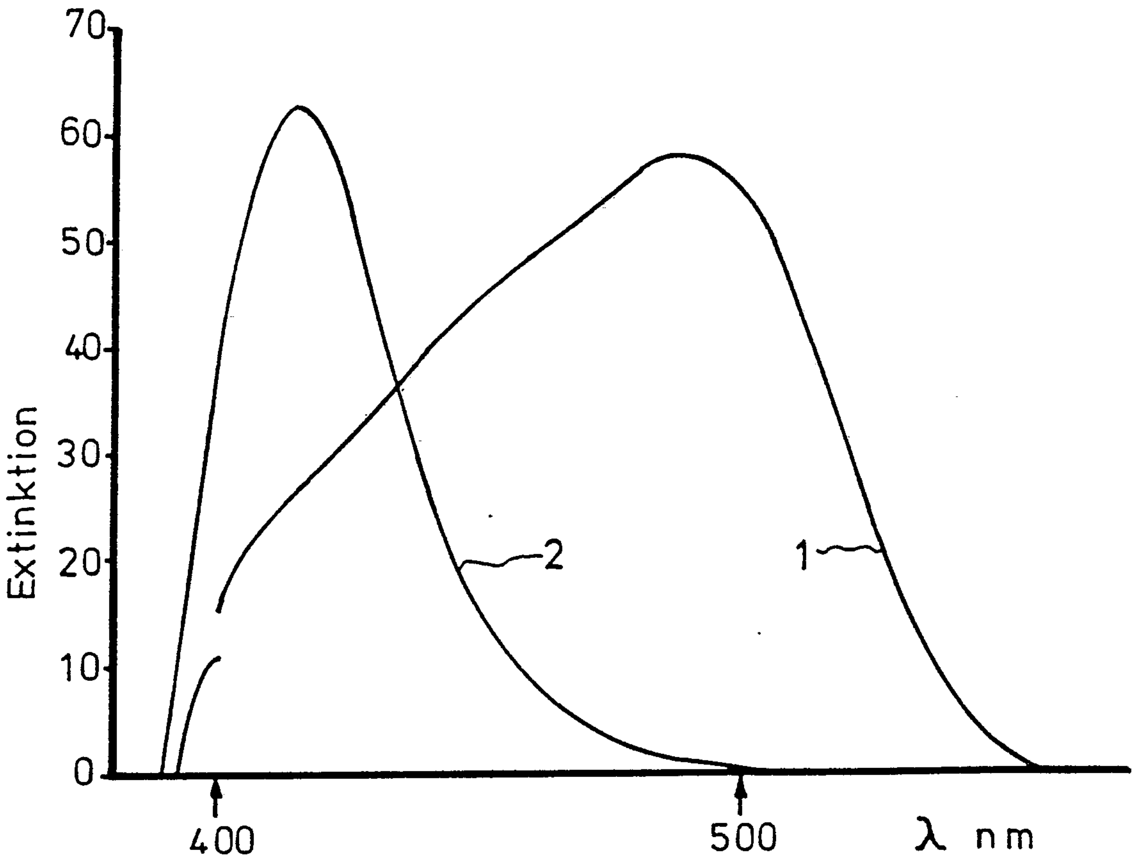
70
60
50
40
30
20
10
0
Extinktion
2
1
400
500
λ nm